# EUROPÄISCHE PATENTSCHRIFT

(11) **EP 1 818 041 B1**
(45) Veröffentlichungstag und Bekanntmachung des Hinweises auf die Patenterteilung: **10.11.2010**
(21) Anmeldenummer: 07004211.4
(22) Anmeldetag: 14.04.2001
(51) Int. Cl.: A61K 47/24, A61K 9/127, A61K 8/04, A61K 8/58, A61K 9/12, A61K 9/48, A61K 8/55, A61Q 19/00

(54) **Pharmazeutische und/oder kosmetische Zubereitung enthaltend ein Organosiloxan und ein Phospholipid**
Pharmaceutical and/or cosmetic composition containing an organosiloxane and a phospholipid
Composition pharmaceutique et/ou cosmétique contenant un organosiloxane et un phospholipide

(30) Priorität: 19.05.2000 DE 10024413
(43) Veröffentlichungstag der Anmeldung: 15.08.2007
(62) Teilanmeldung aus: 01940156.1
(73) Patentinhaber: MIKA Pharma Gesellschaft für die Entwicklung und Vermarktung pharmazeutischer Produkte mbH, 67059 Ludwigshafen (DE)
(72) Erfinder: Piotrowiak, Ralf, 32545 Bad Oeynhausen (DE); Seigfried, Bernd G., 67117 Limburgerhof (DE)
(74) Vertreter: Lau-Loskill, Philipp

(56) Entgegenhaltungen:
- EP-A- 0 834 312
- WO-A-99/38483
- FR-A- 2 777 179
- FR-A- 2 777 181
- US-A- 5 582 815
- US-A- 5 660 853
- PATENT ABSTRACTS OF JAPAN Bd. 018, Nr. 437 (C-1238), 16. August 1994 (1994-08-16) -& JP 06 135816 A (EARTH CHEM CORP LTD), 17. Mai 1994 (1994-05-17)
- BALSAM M S ET AL.: "Cosmetics - Science and Technology" 1979, WILEY INTERSCIENCE , NEW YORK (US) , XP002432351 Seite 32-43
- LIST P H ET AL.: "Arzneiformenlehre" 1985, WISSENSCHAFTLICHE VERLAGSGESELLSCHAFT MBH , STUTTGART (D) , XP002432352 Seite 158-161; Seite 202-204
- ANONYMOUS: "Lecithine" WIKIPEDIA, [Online] Gefunden im Internet: URL:http://de.wikipedia.org/wiki/Lecithin> [gefunden am 2008-09-23]

## Beschreibung

Die vorliegende Erfindung betrifft eine pharmazeutische und/oder kosmetische Zubereitung zur Anwendung beim Menschen, beim Tier oder bei Pflanzen mit den Merkmalen des Oberbegriffs des Patentanspruchs 1.

Kosmetische und/oder pharmazeutische Zubereitungen der eingangs genannten Art sind in unterschiedlichen Darreichungsformen und Formulierungen bekannt. Hierbei weisen diese bekannten Zubereitungen mindestens einen entsprechenden pharmazeutischen und/oder kosmetischen Wirkstoff auf, während andere Inhaltsstoffe der bekannten Zubereitungen dazu dienen, bestimmte, erwünschte Eigenschaften des Wirkstoffes selbst oder der Zubereitung, so zum Beispiel eine spezielle Darreichungsform, zu ermöglichen.

Auch sind pharmazeutischen Zubereitungen zur Anwendung beim Menschen bekannt, wobei diese bekannten Zubereitungen neben mindestens einem pharmazeutischen Wirkstoff desweiteren mindestens eine siliciumorganische Verbindung auf der Basis eines oligomeren und/oder polymeren Diorganosiloxans aufweisen. So beschreibt die US 5,582,815 eine therapeutische oder kosmetische Zubereitung, die topisch in Form eines Aerosols appliziert wird und die neben einem therapeutischen oder kosmetischen Wirkstoff, vorzugsweise neben einem Mittel gegen Pilzbefall, eine hohe Konzentration eines Polydiorganosiloxans aufweist.

Desweiteren ist aus der FR-A 2 777 181 eine kosmetische Zusammensetzung bekannt, die neben nicht näher spezifizierten Lecithinen eine siliciumorganische Verbindung, nämlich Cyclomethicon, enthält, wobei alle durch die Ausführungsbeispiele 1 bis 4 belegten Produkte einen Wassergehalt von etwa 70 Gew.% aufweisen. Die Konzentration an Lecithin variiert, je nach Ausführungsbeispiel zwischen etwa 4 Gew.% und etwa 20 Gew.%, während die Konzentration der siliciumorganischen Verbindung zwischen 9,8 Gew.% und 12 Gew.%, jeweils bezogen auf die anwendungsfertige kosmetische Zusammensetzung, liegt. Auch die FR-A 2 777 179, die auf die selbe Anmelderin wie die zuvor genannte französische Patentanmeldung zurückgeht, beschreibt eine kosmetische Zusammensetzung, die Lecithine und siliciumorganische Verbindungen enthält, wobei diese Schrift bezüglich der Konzentration an Phosphatidylcholin in den Lecithinen eine Angabe dahingehend macht, daß diese Phosphatidylcholin-Konzentration zwischen 22 Gew.% und 29 Gew.% variiert.

In der JP-A 06135816 (Patent Abstracts of Japan, Bd. 018, Nr. 437) wird die Ölkomponente eines Make-ups beschrieben, die in der Lage ist, die übrigen Bestandteile des Make-ups zu lösen. Bei dieser Ölkomponente handelt es sich um eine Mischung eines phospholipidischen Inhaltsstoffes mit einem Silikonpolymer, einem cyclischen Silikon und einem Silikonöl.

Vielfach besteht jedoch das Problem, daß entsprechende pharmazeutische und/oder kosmetische Wirkstoffe nicht oder nur sehr bedingt in dem jeweils eingesetzten Diorganosiloxan löslich oder dispergierbar sind, so daß die Herstellung einer gegen Entmischung stabilen entsprechenden pharmazeutischen und/oder kosmetischen Zubereitung erhebliche Probleme bereitet.

Der vorliegenden Erfindung liegt die Aufgabe zugrunde, eine pharmazeutische und/oder kosmetische Zubereitung zur Anwendung beim Menschen, beim Tier oder bei der Pflanze zur Verfügung zu stellen, die eine besonders hohe Stabilität besitzt.

Diese Aufgabe wird erfindungsgemäß durch eine pharmazeutische und/oder kosmetische Zubereitung der zuvor genannten Art mit dem kennzeichnenden Merkmal des Patentanspruchs 1 gelöst.

Die erfindungsgemäße pharmazeutische und/oder kosmetische Zubereitung zur Anwendung beim Menschen, beim Tier oder bei Pflanzen weist mindestens einen pharmazeutischen bzw. kosmetischen Wirkstoff sowie mindestens eine siliciumorganische Verbindung auf der Basis eines oligomeren und/oder polymeren Diorganosiloxanes auf, wobei die erfindungsgemäße Zubereitung desweiteren mindestens ein Phospholipid enthält. Mit anderen Worten unterscheidet sich somit die erfindungsgemäße Zubereitung von der zuvor in Verbindung mit der US 5,582,815 abgehandelten bekannten Zubereitung dahingehend, daß zusätzlich in der erfindungsgemäßen Zube-reitung mindestens ein spetielles Phospholipid vorhanden ist.

Die erfindungsgemäße Zube-reitung weist als siliciumorganische Verbindung eine cyclische oligomere Siloxan-Verbindung der allgemeinen Formel I

(R₁-SiO-R₂)ₙ Formel I

und/oder eine lineare Siloxan-Verbindung der allgemeinen Formel II 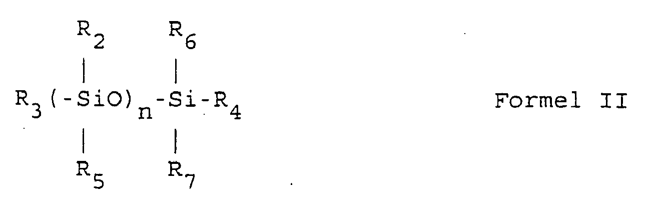 auf, wobei in beiden Formeln I und II

R₁, R₂, R₃, R₄, R₅, R₆ und/oder R₇ jeweils gleich oder verschieden sind und eine C₁-C₄-Alkylgruppe, vorzugsweise eine gesättigte C₁-C₄-Alkylgruppe, und/oder R₃ und/oder R₄, R₆ und/oder R₇ gleich oder verschieden sind und Wasserstoff und/oder eine Hydroxygruppe und n eine ganze Zahl zwischen 0 und 30, vorzugsweise zwischen 0 und 8, bedeuten.

Derartige Siloxane, die auch als flüchtige Silikone bezeichnet und im Handel erhältlich sind, haben eine niedrige Oberflächenspannung, vorzugsweise eine Oberflächenspannung im Bereich zwischen 18 und 21 dyn/cm, sind inert und besitzen ein hohes Netzvermögen, so daß sie in solchen Formulierungen, die topisch appliziert werden, besonders bevorzugt sind. Dies hängt nicht nur damit zusammen, daß diese speziellen cyclischen bzw. linearen Siloxan-Verbindungen aufgrund ihrer Inertheit nicht nur einer hiermit formulierten Zubereitung die erforderliche Stabilität verleihen, sondern auch damit, daß sie sehr schnell nach dem Auftrag verdunsten und darüber hinaus ein gutes Reinigungs- und Lösevermögen für die auf der Hautoberfläche vorhandene Fette bzw. fettähnliche Stoffe und sonstigen Verunreinigungen besitzen, so daß beim Auftragen und ggf. beim leichten Verreiben zusätzlich noch ein Reinigungseffekt auftritt. Darüber hinaus sind diese cyclischen und/oder linearen Siloxane wasserabweisend und gut hautverträglich, wodurch sie insbesondere auch für die topische Anwendung hervorgehoben werden.

Eine gute Stabilität und ein hohes Löse- bzw. Dispergiervermögen, eine sterile Filtrier-barkeit, die leichte Kontrollmöglichkeit auf Fremdparti-kel und die rückfettenden Wirkung der erfindungsgemäßen Zubereitung. Wird dadurch erreicht, daß als Phospholipid mindestens ein an Phosphatidylcholin (1,2-Diacylglycero-3-Phosphatidylcholin) reiches Phospholipid vorhanden ist, wobei in der erfindungsgemäßen Zubereitung solche Phospholipide vorgesehen werden, die mehr als 70 Gew.%, insbesondere mindestens 80 Ges.% und vorzugsweise mehr als 90 Gew.% Phosphatidylcholin, bezogen auf das Gesamtgewicht des jeweils verwendeten Phospholipids, enthalten. Die erfindungsgemäße Zubereitung enthält des weiteren die silicium organische Verbindung in einer Kontentration zwischen 20 Gew % und 85 Gew % und das Phospholipid in einer Konzentration zwischen 5 Gew % und 20 Gew %.

Der bei der erfindungsgemäßen Zubereitung verwendete Begriff pharmazeutische Wirkstoff soll auch solche Wirkstoffe abdecken, die bei Pflanzen eine bestimmte biologische Wirksamkeit besitzen, wie dies nachfolgend noch erläutert ist.

Die zuvor beschriebene erfindungsgemäße Zubereitung weist eine Reihe von Vorteilen auf. So ist zunächst festzuhalten, daß sie eine ausgezeichnete Stabilität besitzt, so daß selbst bei einer längeren Lagerung, insbesondere auch bei erhöhten Temperaturen, keine Entmischung der Inhaltsstoffe, kein Agglomerieren des Wirkstoffes bzw. der Wirkstoffe und auch kein unerwünschter chemischer Abbau des Wirkstoffes bzw. der Wirkstoffe stattfindet. Dies ist um so erstaunlicher, zumal bekannt ist, daß Phospholipide, insbesondere auch Phosphatidylcholin-haltige Phospholipide, bei einer Lagerung zu einem unerwünschten chemischen Abbau neigen, wobei derartige Abbauprodukte dann die Gefahr beinhalten können, daß hierdurch der chemische Abbau des Wirkstoffes bzw. der Wirkstoffe katalysiert wird. Dies tritt jedoch, wie bereits vorstehend beschrieben, überraschenderweise in der erfindungsgemäßen Zusammensetzung nicht auf.

Desweiteren konnte festgestellt werden, daß durch das Vorhandensein des mindestens einen Phospholipids in der erfindungsgemäßen Zubereitung die Löslichkeit bzw. die Dispergierbarkeit des Wirkstoffes bzw. der Wirkstoffe in vielen Fällen erst ermöglicht oder zumindestens erheblich verbessert wird, so daß hierdurch im Vergleich zu solchen bekannten Zubereitungen, die lediglich neben dem Wirkstoff noch die zuvor genannte siliciumorganische Verbindung aufweisen, die Möglichkeiten zur Herstellung von Formulierungen auf eine Vielzahl von Wirkstoffen erweitert wird.

So konnte beispielsweise festgestellt werden, daß die Löslichkeit des Wirkstoffes Clotrimazol in Hexamethyldisiloxan allein kleiner als 0,025 Gew.% ist, während sich dieser Wirkstoff in einer Zubereitung, die 10 Gew.% Phospholipid und 90 Gew.% Hexamethyldisiloxan enthält, mit einer 10-fach höheren Konzentration löst, wobei das hierfür verwendete Phospholipid 25 Gew.% Ethanol und 75 Gew.% Phospholipid aufwies, das 76 ± 3 Gew.% Phosphatidylcholin, 3 ± 3 Gew.% Lysophosphatidylcholin, bis 8 Gew.% Phosphatidsäure, bis 4 Gew.% Phosphatidylethanolamin und maximal 9 Gew.% sonstiger phospholipidische und nicht-phospholipidische Bestandteile, wie insbesondere Öle, Fette und/oder Triglyceride, enthielt.

Außerdem bewirkt der Zusatz des mindestens einen Phospholipids in der erfindungsgemäßen Zubereitung, daß innerhalb von kürzester Zeit die lokal äußerlich aufgetragene Zubereitung unmittelbar in die Haut des Menschen oder Tiers bzw. in die äußeren Pflanzenschichten der hiermit behandelten Pflanzen einzieht, so daß ein unerwünschtes Abreiben einer derartig topisch aufgetragenen erfindungsgemäßen Zubereitung oder ein Abwaschen derselben nicht auftreten kann. Bei einer derartigen Applikation bildet sich innerhalb von wenigen Sekunden zunächst eine gelartige Formulierung auf der Haut aus, so daß die ursprünglich flüssig aufgetragene Zubereitung aufgrund dieser Konsistenzumwandlung in der aufgetragenen Zone fixiert wird und hiernach rasch in das Körperinnere einzieht.

Ferner wirkt die erfindungsgemäße Zubereitung rückfettend, so daß dementsprechend hiermit behandelte äußere Körperbereiche entsprechend gepflegt werden, mit der Folge, daß eine entsprechende Heilung oder Linderung beschleunigt herbeigeführt wird. Hautirritationen, wie sie insbesondere bei empfindlichen Anwendern bei Mensch und Tier bei der Verwendung von bekannten Zubereitungen, die kein Phospholipid enthalten, beobachtet werden konnten, treten bei der Anwendung der erfindungsgemäßen Zubereitung nicht auf. Von daher verursacht die erfindungsgemäße Zubereitung, in der maximal 14 Gew.% Alkohol enthalten ist, bei einer topischen Applikation auf empfindliche oder vorgeschädigte Haut auch keine zusätzlichen Schmerzen, wie das üblicherweise bei den gängigen topischen Formulierungen, die mehr als 14,5 Gew.% Alkohol enthalten, der Fall ist, so daß der entsprechende Anwender die hieraus resultierende Anwendungsbarriere bei der erfindungsgemäßen Zubereitung nicht überwinden muß, wodurch eine ständige und reproduzierbare Anwendung, die letztendlich den Heilungserfolg sicherstellt, gewährleistet wird. Desweiteren wird durch den mindestens einen Phospholipid-Zusatz in der erfindungsgemäßen Zubereitung bei einer Vielzahl von unterschiedlich strukturierten Wirkstoffen sichergestellt, daß transparente Lösungen bzw. transparente feindisperse Systeme entstehen, die einerseits steril filtrierbar sind und andererseits sehr leicht aufgrund ihrer Transparenz auf Fremdpartikel kontrolliert werden können. Diese Kontrollmöglichkeit ist insbesondere dann sehr wichtig, wenn die erfindungsgemäße Zubereitung so formuliert wird, daß sie als Infusion oder Injektion zu applizieren ist.

Insbesondere für solche Ausführungsformen der erfindungsgemäßen Zubereitung, die topisch beim Menschen oder beim Tier angewendet werden, ist eine solche siliciumorganische Verbindung der vorstehend genannten Art in der Zubereitung enthalten, deren Dampfdruck zwischen 1 kPa und 7 kPa, vorzugsweise zwischen 3,5 kPa und 5,7 kPa, und deren Siedepunkt zwischen 15 °C und 150 °C variiert. Hierbei beziehen sich die zuvor wiedergegebenen Werte des Dampfdruckes auf eine Temperatur von 25 °C, während die Angaben zum Siedepunkt auf Normaldruck bezogen sind. Diese Ausführungsformen der erfindungsgemäßen Zubereitung weisen den besonderen Vorteil auf, daß sie sehr schnell, vorzugsweise zwischen 2 Sekunden und 30 Sekunden, nach dem Auftragen auf die jeweiligen Körperbereiche nach innen hin einziehen, so daß auf der Oberfläche keine Rückstände, insbesondere auch keine schmierenden Rückstände, verbleiben. Von daher wird bei dieser Ausgestaltung der erfindungsgemäßen Zubereitung im hohem Maße sichergestellt, daß die topisch applizierte Menge auch tatsächlich innerhalb von kürzester Zeit in den Körper gelangt und daß ein Verschmutzen der Verkleidung ausgeschlossen ist, wodurch eine hohe Anwenderfreundlichkeit der erfindungsgemäßen Zubereitung zur Verfügung gestellt wird.

Als weiterer Vorteil der erfindungsgemäßen Zubereitung, die die zuvor genannten siliciumorganischen Verbindungen enthält, ist festzuhalten, daß derartige siliciumorganische Verbindungen aufgrund ihres schnellen Verdunstens eine Kühlwirkung im Bereich der aufgetragenen erfindungsgemäßen Zubereitung hervorruft, was insbesondere dann als sehr angenehm empfunden wird, wenn die erfindungsgemäße Zubereitung zur Behandlung von entzündlichen Zuständen oder Prellungen oder zur Behandlung von sonstigen Sportverletzungen topisch angewandt wird.

Insbesondere weist die erfindungsgemäße Zubereitung in besonders bevorzugten Ausführungsformen als siliciumorganische Verbindung das cyclische Tetramer der allgemeinen Formel I, wobei in Formel I n gleich 4 und R₁ und R₂ jeweils eine CH₃-Gruppe bedeuten, das cyclische Pentamer der allgemeinen Formel I, wobei in Formel I n gleich 5 und R₁ und R₂ jeweils eine CH₃-Gruppe bedeuten, und/oder ein lineares Siloxan der allgemeinen Formel II auf, wobei in Formel II n gleich 2, 3 oder 4 und R₂ bis R₇ jeweils eine CH₃-Gruppe bedeuten, wobei Hexamethyldisiloxan besonders bevorzugt wird.

Selbstverständlich kann auch in der erfindungsgemäßen Zubereitung ein Gemisch der zuvor beschriebenen Siloxane enthalten sein.

Bezüglich des in der erfindungsgemäßen Zubereitung enthaltenen mindestens einen Phospholipids ist festzuhalten, daß es sich hierbei vorzugsweise um ein solches Phospholipid bzw. Phospholipidgemisch handelt, das aus natürlichen Ausgangsstoffen, so insbesondere Soja, Saflor, Baumwollsaaten, Sonnenblumen und/oder Ei, isoliert ist, wobei derartige Phospholipide vorzugsweise als ölfreie Phospholipide verwendet werden.

Wenn bei einem derartigen, zuvor beschriebenen an Phosphatidylcholin reichem Phospholipid bzw. Phospholipidgemisch zusätzlich noch dessen Acylreste in Position 1 und in Position 2 zu 10 bis 15 Gew.% aus dem Palmitinsäurerest, zu 1,4 bis 4 Gew.% aus dem Stearinsäurerest, zu 3 bis 10 Gew.% aus dem Ölsäurerest, zu 61 bis 71 Gew.% aus dem Linolsäurerest und zu 3 bis 7 Gew.% aus dem Linolensäurerest, bezogen auf die Summe aller Acylreste in Position 1 und 2, bestehen, verleiht ein derartiges Phosphatidylcholin in Verbindung mit den zuvor beschriebenen siliciumorganischen Verbindungen der erfindungsgemäßen Zubereitung eine hohe chemische Stabilität, so daß ein unerwünschter Abbau des Wirkstoffes bzw. des Wirkstoffgemisches selbst bei einer mehrmonatigen Lagerung unter extremen Bedingungen nicht oder nur im völlig untergeordnetem Maße stattfindet.

Bei einer anderen Ausgestaltung der erfindungsgemäßen Zubereitung weist diese als Phospholipid ein hydriertes Phospholipid, vorzugsweise ein hydriertes Phosphatidylcholin, auf. Derartige hydrierte Phospholipide erleichtern desweiteren die Herstellung von transparenten Zubereitungen, so daß hier nur ein kurzes Vermischen der Inhaltsstoffe der erfindungsgemäßen Zubereitung erforderlich ist, um entsprechend steril filtrierbare und transparente Lösungen bzw. Feindispersionen des Wirkstoffes zu erstellen, die dann entsprechend, ggf. nach Sterilfiltration durch ein 0,2 µm Filter und/oder nach zusätzlicher Sterilisation, dann auch sicher durch Injektion oder Infusion dargereicht werden können.

Durch Variation und Abstimmung der zuvor beschriebenen siliciumorganischen Verbindung und des zuvor beschriebenen mindestens einen Phospholipids in Kombination mit Wasser lassen sich auch Ausführungsformen der erfindungsgemäßen Zubereitung formulieren, deren Wassergehalt zwischen 0 Gew.% und 5 Gew.% variiert und bei denen die in der Zubereitung enthaltene siliciumorganische Verbindung und das Phospholipid derart miteinander vermischt sind, daß die Zubereitung bei Kontakt mit Wasser spontan Liposome und/oder Mizellen ausbildet. Hierbei zeigte sich, daß überraschenderweise bereits die geringe, in der Haut und insbesondere in den oberen Hautschichten natürlicherweise vorkommende Wasserkonzentration ausreicht, um auf der Hautoberfläche spontan diese Liposome und/oder Mizellen auszubilden, so daß eine derartige Weiterbildung der erfindungsgemäßen Zubereitung insbesondere für die topische Anwendung bevorzugt wird.

Vorzugsweise werden bei der zuvor beschriebenen Ausgestaltung der erfindungsgemäßen Zubereitung die siliciumorganischen Verbindungen, das Phospholipid und das Wasser derart aufeinander abgestimmt, daß die sich durch Zugabe von Wasser spontan bildenden Liposome bzw. Mizellen eine mittlere Teilchengröße zwischen 50 nm und 4.000 nm, insbesondere zwischen 250 nm und 1.500 nm, aufweisen, so daß derartige Liposome bzw. Mizellen besonders geeignet sind, die in der erfindungsgemäßen Zubereitung enthaltenen Wirkstoffe bei einer topischen Applikation schnell und vollständig in das Innere des menschlichen oder tierischen Körpers oder in das Innere der Pflanze zu transportieren. Hierdurch wird insbesondere auch sichergestellt, daß der jeweilige Wirkstoff schnell und zerstörungsfrei dort hingelangt, wo er die diesbezügliche kosmetische bzw. pharmazeutische Wirksamkeit zeigen soll.

Abhängig von der jeweiligen Verwendung der erfindungsgemäßen Zubereitung und deren Darreichungsform weist die erfindungsgemäße Zubereitung neben der mindestens einen siliciumorganischen Verbindung der eingangs beschriebenen Art und dem Phospholipid ggf. desweiteren Wasser, Alkohol, Antioxidantien, nicht phospholipidische Emulgatoren, pharmazeutische Wirkstoffe, kosmetische Wirkstoffe und/oder weitere Hilfsstoffe, wie beispielsweise Gelbildner, Verdickungsmittel, Duftstoffe, Konservierungsmittel o.dgl., auf.

Insbesondere variiert bei der erfindungsgemäßen Zubereitung die Konzentration des mindestens einen pharmazeutischen Wirkstoffes und/oder des mindestens einen kosmetischen Wirkstoffes in der Zubereitung zwischen 0,001 Gew.% und 30 Gew.%, vorzugsweise zwischen 0,1 Gew.% und 15 Gew.%, jeweils bezogen auf die anwendungsfertige Zubereitung.

Eine besonders geeignete Ausführungsform der erfindungsgemäßen Zubereitung, die vorzugsweise für die topische Applikation verwendet wird, sieht vor, daß die Zubereitung desweiteren mindestens einen Alkohol, insbesondere Ethanol, Propanol-1 und/oder Propanol-2, enthält, wobei die Konzentration des mindestens einen Alkohols maximal 14 Gew.% beträgt und vorzugsweise zwischen 0,1 Gew.% und 8,5 Gew.% variiert, jeweils bezogen auf die anwendungsfertige Zubereitung. Diese Ausgestaltung der erfindungsgemäßen Zubereitung weist aufgrund des Alkoholgehaltes den zusätzlichen Vorteil auf, daß diese Ausführungsform der erfindungsgemäßen Zubereitung von vornherein schon steril ist, so daß die Anwendung von zusätzlichen Konservierungsmitteln entfallen kann. Außerdem konnte überraschend festgestellt werden, daß selbst bei hoher Empfindlichkeit des jeweiligen Anwenders gegenüber Alkohol keine Hautirritationen auftraten, sofern die Alkoholkonzentration in der erfindungsgemäßen Zubereitung auf maximal 14 Gew.% begrenzt wird. Dies wird darauf zurückgeführt, daß die erfindungsgemäße Zubereitung als wesentliche Inhaltsstoffe die Kombination von siliciumorganischer Verbindung mit Phospholipid enthält, so daß durch diese hautheilende und hautschonende Kombination die negativen Hauteinflüsse des Alkohols ausgeschlossen werden. Selbst wenn man eine derartige Ausgestaltung der erfindungsgemäßen Zubereitung auf hochentzündliche Hautbereich topisch oder auf hochentzündliche Schleimhautbereiche aufträgt, konnten keine negativen Nebeneffekte, wie beispielsweise ein unangenehmes und schmerzhaftes Brennen, festgestellt werden.

Bezüglich des in der erfindungsgemäßen Zubereitung enthaltenen mindestens einen Wirkstoffs ist festzuhalten, daß sich zunächst die Auswahl des Wirkstoffes danach richtet, ob die erfindungsgemäße Zubereitung beim Menschen, beim Tier oder bei der Pflanze angewendet werden soll.

Für die menschliche Anwendung oder für die Anwendung beim Tier weist die erfindungsgemäße Zubereitung insbesondere einen solchen pharmazeutischen Wirkstoff auf, der aus der Gruppe ausgewählt wird, die Lokalanästhetika, Antiallergika, Dermatika, Wirkstoffe gegen grippale Infekte und Erkältungskrankheiten, Wirkstoffe zur Behandlung von Neuropathien, Wirkstoffe zur Behandlung von Durchblutungsstörungen, Chemotherapeutika, Chinin, Antimykotika, Antibiotika, Thalidomid, Analgetika, Nicht-steroidale Antirheumatika, Leukotriene, Leukotrienhemmer, Antiandrogene, Kortikoide, Opiatrezeptor-Antagonisten, Blutgerinnung hemmende Stoffe, Thrombozytenaggregationshemmer, Histaminantagonisten, regulatorisch und enzymatisch wirkende Peptide und Proteine, Nukleinsäuren und Oligopeptide, Antidiabetika, Prostaglandine, Prostaglandinsynthesehemmer, Antiviral wirkende oder virostatisch wirkende Substanzen, Antimikrobiell-wirkende Substanzen, Wirkstoffe gegen Prione, Immunsupressiva, Hormone, Wirkstoffe zur Behandlung von Wunden und insbesondere chronischen Wunden, Vitamine, Pflanzenextrakte oder Auszüge aus Pflanzenextrakten, Psychopharmaka, den Schlaf beeinflussende Wirkstoffe, Analeptika, Allgemeinanästhetika, Muskelrelaxantien, Antiepileptika, Antiparkinsonmittel, Antiemetika, Ganglionär angreifende Substanzen, am Symphatikus angreifende Substanzen, am Parasamphatikus angreifende Substanzen, Calciumantagonisten, Herz/Kreislaufmittel, Antiasthmatika, Antitussiva, Expektorantien, Hepatika, Diuretika, Choleretika, Desinfektionsmittel, Spurenelemente, Antiinfaktiva, Zytostatika, Antimetaboliten, Hormonanatgonisten, Immunmodulatoren und/oder Wirkstoffe gegen innere oder äußere Parasiten umfaßt.

Wird hingegen die erfindungsgemäße Zubereitung im Pflanzenbereich verwendet, so ist bei dieser speziellen Ausgestaltung der erfindungsgemäßen Zubereitung als Wirkstoff ein Fungizid, ein Insektizid, ein Herbizid und/oder ein über Blatt- und/oder oberirdischen Pflanzenteilen aufnehmbares Düngemittel vorhanden.

Wie bereits zuvor wiederholt herausgestellt wurde, wird die erfindungsgemäße Zubereitung vorzugsweise auch topisch appliziert, wobei hierfür alle möglichen topischen Applikationsdarreichungsformen, so zum Beispiel eine topische Applikation als Gel, als Creme, als Spray, als Puder oder als Pflaster, denkbar sind.

Insbesondere weist die erfindungsgemäße Zubereitung für die topische Applikation eine flüssige bis pastöse Viskosität auf, wobei die Viskosität der Zubereitung vorzugsweise zwischen 0,4 cSt und 10.000 cSt, insbesondere zwischen 0,6 cSt und 1.500 cSt, variiert. Die in der vorliegenden Anmeldung verwendeten Viskositätsangaben werden alle bei 25 °C bestimmt. Diese flüssige bis pastöse Darreichungsform der erfindungsgemäßen Zubereitung läßt sich einerseits sehr leicht auftragen und zieht andererseits sehr schnell ins Körperinnere ein, wodurch die Handhabung und die Effektivität der erfindungsgemäßen Zubereitung sichergestellt wird.

Insbesondere wird bei der zuvor beschriebenen topischen Applikation der erfindungsgemäßen Zubereitung die mindestens eine siliciumorganische Verbindung und das mindestens eine Phospholipid sowie ggf. ein Lösungsmittel, vorzugsweise ein Alkohol, bezüglich ihrer Massenverhältnisse derart aufeinander abgestimmt, daß nach Auftragen der Zubereitung auf die menschliche bzw. tierische Haut in einer Konzentration zwischen 0,05 g und 0,3 g, jeweils pro 100 cm² Haut, die so aufgetragene Zubereitung innerhalb einer Zeit von 1 Sekunde bis 60 Sekunden, vorzugsweise innerhalb einer Zeit von 3 Sekunden bis 20 Sekunden, keine sichtbaren Rückstände mehr auf der Haut verbleiben. Hierdurch wird dann verhindert, daß eine Beschmutzung der Kleidung und/oder ein unerwünschtes Abreiben bzw. Abstreifen der so topisch aufgetragenen Zubereitung herbeigeführt wird, wodurch eine leichte und reproduzierbare Anwendung der erfindungsgemäßen Zubereitung sichergestellt wird.

Zusätzlich zu den zuvor genannten Wirkstoffen oder anstelle der zuvor genannten Wirkstoffe weist eine weitere Ausführungsform der erfindungsgemäßen Zubereitung mindestens ein Vitamin und insbesondere Vitamin C, Vitamin E, Vitamin A und/oder Pro-Vitamin A auf, wobei diese Vitamine wahlweise als pharmazeutische und/oder kosmetische Wirkstoffe aufzufassen sind.

Wie bereits vorstehend wiederholt dargelegt ist, kann die erfindungsgemäße Zubereitung neben der topischen Applikation auch in jeder anderen Darreichungsform aufgemacht sein, wobei eine Gabe der erfindungsgemäßen Zubereitung als Kapsel, Tablette, Zäpfchen, Pflaster, Spray, Nebel, Gel, Puder und/oder Creme erfolgen kann. Neben diesen speziellen Darreichungsformen und/oder der besonders bevorzugten topischen Anwendung kann die erfindungsgemäße Zubereitung ebensogut buccal, nasal, oral, anal, per Injektion und/oder Infusion dargereicht werden.

Vorteilhafte Weiterbildungen der erfindungsgemäßen Zubereitung sind in den Unteransprüchen angegeben.

Bezüglich des im vorliegenden Text verwendeten Begriffs Wasser ist festzuhalten, daß dieser Begriff Wasser nicht nur Wasser selbst, d.h. destilliertes und/oder entionisiertes Wasser, sondern auch alle übrigen wäßrigen Systeme, so insbesondere Salz- oder Pufferlösungen, vorzugsweise Phosphat-, Citrat- und/oder Acetatpuffer, abdeckt.

Alkohol im Sinne der vorliegenden Beschreibung deckt alle aliphatischen Alkohole mit einer Kohlenstoffkettenlänge von 1 bis 30 sowie mit 1 bis 10 Hydroxyl-Gruppen ab, vorzugsweise jedoch die niedermolekularen einwertigen C₂-C₅-Alkohole sowie die entsprechenden zweiwertigen Glykole.

Die erfindungsgemäße Zubereitung wird nachfolgend anhand von Ausführungsbeispielen im Detail näher erläutert.

### Ausführungsbeispiel 1

sprühbare Gelformulierung mit dem Wirkstoff Clotrimazol

Es wurde eine sprühbare Gelformulierung hergestellt, die die folgenden Inhaltsstoffe aufwies:

| | |
|---|---|
| Clotrimazol | 1 Gew.% |
| Propanol-2 | 8 Gew.% |
| Hexamethyldisiloxan | 78,8 Gew.% |
| Phospholipid (75 Gew.% Phosphatidylcholin, 25 Gew.% Ethanol) | 10 Gew.% |
| Pfefferminzöl | 0,2 Gew.% |
| 1,2-Propanidol | 2 Gew.% |

Zur Herstellung dieser Formulierung wurde der zuvor genannte kosmetische Wirkstoff vorgelegt und in dem Phospholipid, das 75 Gew.% Phosphatidylcholin und 25 Gew.% Ethanol aufwies, unter Zusatz etwa der Hälfte des zuvor ausgewiesenen Hexamethyldisiloxan unter Rühren bei Raumtemperatur gelöst. Nachdem eine klare Lösung resultierte, wurde unter Rühren bei Raumtemperatur die verbleibende Teilmenge Hexamethyldisiloxan zugesetzt.

Die so hergestellte sprühbare Formulierung, die gute Gelund Filmbildungseigenschaften besaß und darüber hinaus noch stark benetzend war, wurde mit 2 Sprühstößen (jeweils 0,2 ml aus einem mechanischem Sprühsystem, Braunglasflasche verschlossen mit Mistette MARK II Sprühkopf) auf den Handrücken aus 20 cm Entfernung aufgetragen. Es bildete sich hierbei ein gleichmäßig verteilter homogener Film aus, der innerhalb von 60 Sekunden komplett ohne Hinterlassen eines Rückstandes in die Haut eingezogen bzw. verdunstet war.

Zur Bestimmung der Stabilität wurde der Wirkstoffgehalt direkt nach der Herstellung gemessen. Nach einer Lagerung von 60 Tagen bei 40 °C und einer Lagerung von 120 Tagen, ebenfalls bei 40 °C, konnte kein Verlust an Wirkstoff festgestellt werden. Auch konnte das Hauptabbauprodukt des Clotrimazols, d.h. das Carbinol, nach entsprechender Lagerung nicht detektiert werden.

Desweiteren wurden Lagerungsversuche durchgeführt, wobei jeweils eine Probe der zuvor genannten Sprühformulierung drei Monate bei 4 °C, drei Monate bei 25 °C und drei Monate bei 40 °C gelagert wurde. Bei keiner der gelagerten Proben trat eine Phasentrennung oder eine Kristallbildung auf. Alle gelagerten Proben waren, wie die Ausgangsprobe, dünnflüssig, leicht gelb gefärbt, transparent und sprühbar.

Die vorstehend beschriebene Sprühformulierung war darüber hinaus auf Fremdpartikel kontrollierbar, ließ sich steril filtrieren (0,2 µm Filter) und konnte durch einfaches Rühren ohne Homogenisieren und ohne zusätzliche Temperaturerhöhung bzw. Druckänderungen hergestellt werden.

Zum Nachweis der pharmazeutischen Anwendung der antimykotischen Sprühformulierung, die eine hohe Wirksamkeit bei allen Hautmykosen, so insbesondere bei Fußpilz, bei Vaginalmykosen und/oder Nagelmykosen, aufweist, die durch eine äußerliche Behandlung zugänglich sind, wurde die zuvor beschriebene und nach Ausführungsbeispiel 1 hergestellte Sprühformulierung im Vergleich zu einem herkömmlichen Handelsprodukt, das ebenfalls 1 Gew.% Clotrimazol als Wirkstoff in einer Formulierung, die zu mehr als 50 % aus Isopropanol zusammengesetzt war und ferner Macrogol 400 sowie Propylenglykol enthielt, bei 46 gesunden Probanden (zweimal 23 Probanden pro Gruppe) eine verblindete Verträglichkeitsstudie (dreimal täglich drei Sprühstöße ä 0,2 ml auf ca. 100 cm² Haut im Bauchbereich) über einen Behandlungszeitraum von 10 Tagen durchgeführt.

Als Ergebnis dieser Studie ist festzuhalten, daß bei keinem der Probanden, die mit der Sprühformulierung gemäß Ausführungsbeispiel 1 behandelt wurden, eine Hautreaktion feststellbar war. Auch trat keine Austrocknung oder Unverträglichkeit im besprühten Bereich auf.

Sechs der Probanden, die mit dem herkömmlichen Produkt behandelt wurden, zeigten im behandelten Bereich der Bauchhaut im Vergleich zu der benachbarten unbehandelten Haut deutlich erkennbare Austrocknungen. Drei Probanden davon wiesen zusätzlich zur Hautaustrocknung noch Hautirritationen in Form von Hautrötungen und Pustelbildungen auf.

### Ausführungsbeispiel 2

Es wurden drei unterschiedliche, topisch applizierbare Formulierungen des Wirkstoffes Acetylsalicylsäure erstellt, wobei hier zur Herstellung dieser Formulierungen so vorgegangen wurde, wie dies vorstehend bei Ausführungsbeispiel 1 beschrieben ist. Die diesbezüglichen Formulierungen wiesen folgende Inhaltsstoffe auf:

**Formulierung A**

| | |
|---|---|
| Acetylsalicylsäure | 6,95 Gew.% |
| Phospholipid | 16,2 Gew.% |
| Isopropanol | 22,2 Gew.% |
| Octamethylcylcotetrasiloxan | 54,65 Gew.% |

**Formulierung B**

| | |
|---|---|
| Acetylsalicylsäure | 5,2 Gew.% |
| Phospholipid | 12,1 Gew.% |
| Isopropanol | 16,6 Gew.% |
| Hexamethyldisiloxan | 66,1 Gew.% |

**Formulierung C**

| | |
|---|---|
| Acetylsalicylsäure | 3 Gew.% |
| Phospholipid | 7,2 Gew.% |
| Isopropanol | 9,8 Gew.% |
| Hexamethyldisiloxan | 80 Gew.% |

In den zuvor genannten Formulierungen A bis C wurde als Phospholipid eine Lösung von 75 Gew.% Phosphatidylcholin in 25 Gew.% Ethanol verwendet.

Die zuvor beschriebenen Formulierungen A bis C dienen zur topischen und lokalen epikutanen Anwendung, insbesondere im Bereich der Schmerzbekämpfung (Kopfschmerzen, Muskelschmerzen, Rheuma) und zur Blutverdünnung. Diese Formulierungen waren auch nach einer Lagerung von über drei Monaten bei einer Lagertemperatur von 40 °C stabil.

### Ausführungsbeispiel 3

Es wurde eine flüssige Zubereitung zur Behandlung von Ekzemen, wie insbesondere endogene, toxisch-degenerative und seborr. Ekzeme, allerg. Kontaktekzeme, Stauungsekzeme, entzündliche und allerg. Hauterkrankungen, Psoriasis, Sonnenbrand, Alopezia Areata aus folgenden Inhaltsstoffen erstellt:

| | |
|---|---|
| Hydrocortison rein | 0,1 Gew.% |
| Hexamethyldisiloxan | 65,4 Gew.% |
| Propanol-2 reinst | 5 Gew.% |
| Phospholipid | 8 Gew.% |
| Silmogen Carrier (Handelsprodukt der Firma Dow Corning) | 21,3 Gew.% |
| Pfefferminzöl | 0,15 Gew.% |

Die zuvor genannte wasserfreie sprühbare Zubereitung bildete beim Auftreffen auf die Haut spontan Liposome aus, deren mittlere Partikelgröße im Bereich von 194 nm lag.

### Ausführungsbeispiel 4

Es wurde eine flüssige Zubereitung zur Behandlung von Ekzemen, wie insbesondere endogene, toxisch-degenerative und seborr. Ekzeme, allerg. Kontaktekzeme, Stauungsekzeme, entzündliche und allerg. Hauterkrankungen, Psoriasis, Sonnenbrand, Alopezia Areata aus folgenden Inhaltsstoffen erstellt:

| | |
|---|---|
| Hydrocortison rein | 0,1 Gew.% |
| Hexamethyldisiloxan | 86,7 Gew.% |
| Propanol-2 reinst | 5 Gew.% |
| Phospholipid | 8 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 205 nm lag.

### Ausführungsbeispiel 5

Es wurde eine flüssige, sprühbare Zubereitung zur Behandlung von Dermatomykosen, verursacht durch Dermatophyten, Hefen (z.B. Candida-Arten), Schimmelpilze und andere Pilze, mit entzündlichen bzw. ekzematösen Hauterscheinungen und/oder Juckreiz aus folgenden Inhaltsstoffen erstellt:

| | |
|---|---|
| Clotrimazol | 0,8 Gew.% |
| Propanol-2 | 11,2 Gew.% |
| Hexamethyldisiloxan | 77,8 Gew.% |
| Phospholipid | 10 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 170 nm lag.

### Ausführungsbeispiel 6

Es wurde eine flüssige, sprühbare Zubereitung zur Behandlung bei Sport- bzw. Unfallverletzungen, Weichteilrheumatismus, bei Schmerzen des Bewegungsapparates an Gelenken, Muskeln und/oder Sehnen, die durch unterschiedliche Unfälle, Überlastungen oder sonstigen Erkrankungen ausgelöst werden, aus folgenden Inhaltsstoffen erstellt:

| | |
|---|---|
| Dexibuprofen | 8,3 Gew.% |
| Propanol-2 | 9 Gew.% |
| Hexamethyldisiloxan | 70,2 Gew.% |
| Phospholipid | 12,3 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 105 nm lag.

### Ausführungsbeispiel 7

Es wurde eine flüssige, sprühbare Zubereitung zur Behandlung bei Sport- bzw. Unfallverletzungen, Weichteilrheumatismus, bei Schmerzen des Bewegungsapparates an Gelenken, Muskeln und/oder Sehnen, die durch unterschiedliche Unfälle, Überlastungen oder sonstigen Erkrankungen ausgelöst werden, aus folgenden Inhaltsstoffen erstellt:

| | |
|---|---|
| Ibuprofen | 8,3 Gew.% |
| Propanol-2 | 9 Gew.% |
| Hexamethyldisiloxan | 70,2 Gew.% |
| Phospholipid | 12,3 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 135 nm lag.

### Ausführungsbeispiel 8

Es wurde eine flüssige, sprühbare Zubereitung zur Behandlung von endogenem Ekzem (atopische Dermatitis, Neurodermitis), degenerativem dyshidrotischem vulgärem Ekzem, Kontaktekzem, Neurodermitis aus folgenden Inhaltsstoffen erstellt:

| | |
|---|---|
| Prednisolon | 0,1 Gew.% |
| Propanol-2 | 5 Gew.% |
| Hexamethyldisiloxan | 86,7 Gew.% |
| Phospholipid | 8 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 400 nm lag.

### Ausführungsbeispiel 9

Es wurde eine flüssige, sprühbare Zubereitung zur Behandlung von akuten und chronischen Ekzemen, atopischen Ekzemen (Neurodermitis), Psoriasis, Verbrennungen 1. Grades aus folgenden Inhaltsstoffen erstellt:

| | |
|---|---|
| Prednicarbat | 0,1 Gew.% |
| Propanol-2 | 5 Gew.% |
| Hexamethyldisiloxan | 86,7 Gew.% |
| Phospholipid | 8 Gew.% |
| Pfefferminzöl | 0,2 Gew.% |

Die zuvor genannte wasserfreie sprühbare Zubereitung bildete beim Auftreffen auf die Haut spontan Liposome aus, deren mittlere Partikelgröße im Bereich von 295 nm lag.

Die in den Beispielen 1 und 2 sowie 3 bis 7 jeweils eingesetzten Phospholipide wiesen 25 Gew.% Ethanol und 75 Gew.% Phospholipid auf, das 76 ± 3 Gew.% Phosphatidylcholin, 3 ± 3 Gew.% Lysophosphatidylcholin, bis 8 Gew.% Phosphatidsäure, bis 4 Gew.% Phosphatidylethanolamin und maximal 9 Gew.% sonstiger phospholipidische und nichtphospholipidische Bestandteile, wie insbesondere Öle, Fette und/oder Triglyceride, enthielt.

Das in den Beispielen 8 und 9 jeweils eingesetzte Phospholipid wies 15 Gew.% Isopropanol und 85 Gew.% Phospholipid auf, das 76 ± 3 Gew.% Phosphatidylcholin, 3 ± 3 Gew.% Lysophosphatidylcholin, bis 8 Gew.% Phosphatidsäure, bis 4 Gew.% Phosphatidylethanolamin und maximal 9 Gew.% sonstiger phospholipidische und nicht-phospholipidische Bestandteile, wie insbesondere Öle, Fette und/oder Triglyceride, enthielt.

Zur Herstellung der in dem Beispiel 5 beschriebenen Zubereitungen wurde so vorgegangen, wie dies beim Ausführungsbeispiel 1 vorstehend dargelegt ist.

Um die in den Ausführungsbeispielen 2 bis 4 und 6 bis 9 beschriebenen Zubereitungen zu erstellen, wurde der jeweilige Wirkstoff vorgelegt und in einer Phase, die das Phospholipid sowie ggf. die weiteren Hilfsstoffe (Alkohol, Emulgator, Aromen etc.) enthielt, bei Raumtemperatur unter Rühren gelöst. Nachdem eine vollständige und transparente Lösung erstellt war, erfolgte der Zusatz des Siloxans unter weiterem Rühren bei Raumtemperatur.

Um die in den Ausführungsbeispielen 1 und 5 beschriebenen Zubereitung zu erstellen, wurde der jeweilige Wirkstoff vorgelegt und in einer Phase, die das Phospholipid, ggf. die weiteren Hilfsstoffe (Alkohol, Emulgator, Aromen etc.) und zwischen 10 Gew.% und 40 Gew.% der jeweiligen Gesamtmenge des eingesetzten Siloxans enthielt, bei Raumtemperatur unter Rühren gelöst. Nachdem eine vollständige und transparente Lösung erstellt war, erfolgte der Zusatz der Restmenge des Siloxans unter weiterem Rühren bei Raumtemperatur.

## Patentansprüche

1. Pharmazeutische und/oder kosmetische Zubereitung zur Anwendung beim Menschen, beim Tier oder bei Pflanzen mit mindestens einem pharmazeutischen und/oder kosmetischen Wirkstoff, mit mindestens einem Phospholipid sowie mit mindestens einer siliciumorganischen Verbindung auf der Basis eines oligomeren und/oder polymeren Diorganosiloxanes, **dadurch gekennzeichnet,**
a) **daß** die Zubereitung zwischen 20 Gew.% und 85 Gew.% der siliciumorganischen Verbindung und zwischen 5 Gew.% und 20 Gew.% des mindestens einen Phospholipids enthält,
b) **daß** die Zubereitung als siliciumorganische Verbindung eine cyclische oligomere Siloxan-Verbindung der allgemeinen Formel I
(R₁-SiO-R₂)ₙ Formel I
und/oder eine lineare Siloxan-Verbindung der allgemeinen Formel II 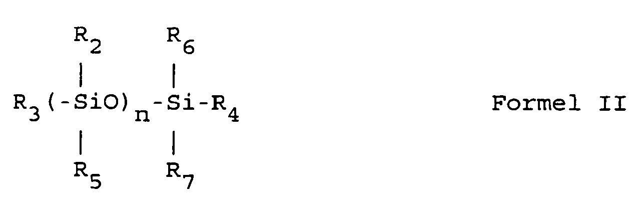 aufweist, wobei in beiden Formeln I und II
R₁, R₂, R₃, R₄, R₅, R₆ und/oder R₇ jeweils gleich oder verschieden sind und eine C₁-C₄-Alkylgruppe, vorzugsweise eine gesättigte C₁-C₄-Alkylgruppe, und/oder
R₃ und/oder R₄, R₆ und/oder R₇ gleich oder verschieden sind und Wasserstoff und/oder eine Hydroxygruppe und
n eine ganze Zahl zwischen 0 und 30 bedeuten, und daß
c) die Zubereitung ein solches Phospholipid aufweist, das mehr als 70 Gew.% Phosphatidylcholin besitzt.

2. Zubereitung nach Anspruch 1, **dadurch gekennzeichnet, daß** n eine ganze Zahl zwischen 0 und 8 bedeutet.

3. Zubereitung nach einem der vorangehenden Ansprüche, **dadurch gekennzeichnet, daß** die Zubereitung eine solche siliciumorganische Verbindung aufweist, deren Dampfdruck zwischen 1 kPa und 7 kPa, vorzugsweise zwischen 3,5 kPa und 5,7 kPa, und deren Siedepunkt zwischen 15 °C und 150 °C variiert.

4. Zubereitung nach Anspruch 1, **dadurch gekennzeichnet, daß** die Zubereitung als siliciumorganische Verbindung das cyclische Tetramer der allgemeinen Formel I, wobei in Formel I n gleich 4 und R₁ und R₂ jeweils eine CH₃-Gruppe bedeuten,
das cyclische Pentamer der allgemeinen Formel I, wobei in Formel I n gleich 5 und R₁ und R₂ jeweils eine CH₃-Gruppe bedeuten,
und/oder
ein lineares Siloxan der allgemeinen Formel II aufweist, wobei in Formel II n gleich 2, 3 oder 4 und R₂, R₃, R₄, R₅, R₆ und R₇ jeweils eine CH₃-Gruppe bedeuten.

5. Zubereitung nach einem der vorangehenden Ansprüche, **dadurch gekennzeichnet, daß** die Zubereitung als Phospholipid ein aus natürlichen Ausgangsstoffen isoliertes Phospholipid aufweist.

6. Zubereitung nach Anspruch 5, **dadurch gekennzeichnet, daß** das Phospholipid mindestens 80 Gew.% und vorzugsweise mehr als 90 Gew.% Phosphatidylcholin enthält.

7. Zubereitung nach einem der vorangehenden Ansprüche, **dadurch gekennzeichnet, daß** das Phospholipid ein hydriertes Phospholipid, vorzugsweise ein hydriertes Phosphatidylcholin, ist.

8. Zubereitung nach einem der vorangehenden Ansprüche, **dadurch gekennzeichnet, daß** die Zubereitung zwischen 0 Gew.% und 5 Gew.% Wasser aufweist und daß die in der Zubereitung enthaltene siliciumorganische Verbindung und das Phospholipid derart miteinander vermischt sind, daß die Zubereitung bei Kontakt mit Wasser spontan Liposome und/oder Mizellen ausbildet.

9. Zubereitung nach Anspruch 8, **dadurch gekennzeichnet, daß** die durch Zugabe von Wasser sich spontan bildenden Liposome bzw. Mizellen eine mittlere Teilchengröße zwischen 50 nm und 4.000 nm, insbesondere zwischen 250 nm und 1.500 nm, aufweisen.

10. Zubereitung nach einem der vorangehenden Ansprüche, **dadurch gekennzeichnet, daß** die Zubereitung neben der mindestens einen siliciumorganischen Verbindung und dem mindestens einen Phospholipid desweiteren Wasser, Alkohol, Antioxidantien, nicht phospholipidische Emulgatoren, pharmazeutische Wirkstoffe, kosmetische Wirkstoffe und/oder weitere Hilfsstoffe aufweist.

11. Zubereitung nach Anspruch 10, **dadurch gekennzeichnet, daß** die Konzentration des mindestens einen pharmazeutischen Wirkstoffes und/oder des mindestens einen kosmetischen Wirkstoffes in der Zubereitung zwischen 0,001 Gew.% und 30 Gew.%, insbesondere zwischen 0,1 Gew.% und 15 Gew.% bezogen auf die anwendungsfertige Zubereitung, variiert.

12. Zubereitung nach einem der vorangehenden Ansprüche, **dadurch gekennzeichnet, daß** die Zubereitung desweiteren mindestens einen Alkohol enthält, wobei die Konzentration des mindestens einen Alkohols maximal 14 Gew.% beträgt und vorzugsweise zwischen 0,1 Gew.% und 8,5 Gew.% variiert, jeweils bezogen auf die anwendungsfertige Zubereitung.

13. Zubereitung nach einem der vorangehenden Ansprüche, **dadurch gekennzeichnet, daß** die Zubereitung zur Anwendung beim Menschen oder Tier mindestens einen kosmetischen und/oder pharmazeutischen Wirkstoff aufweist, der aus der Gruppe ausgewählt ist, die Lokalanästhetika, Antiallergika, Dermatika, Wirkstoffe gegen grippale Infekte und Erkältungskrankheiten, Wirkstoffe zur Behandlung von Neuropathien, Wirkstoffe zur Behandlung von Durchblutungsstörungen, Chemotherapeutika, Chinin, Antimykotika, Antibiotika, Thalidomid, Analgetika, Nicht-steroidale Antirheumatika, Leukotriene, Leukotrienhemmer, Antiandrogene, Kortikoide, Opiatrezeptor-Antagonisten, Blutgerinnung hemmende Stoffe, Thrombozytenaggregationshemmer, Histaminantagonisten, regulatorisch und enzymatisch wirkende Peptide und Proteine, Nukleinsäuren und Oligopeptide, Antidiabetika, Prostaglandine, Prostaglandinsynthesehemmer, Antiviral wirkende oder virostatisch wirkende Substanzen, Antimikrobiell-wirkende Substanzen, Wirkstoffe gegen Prione, Immunsupressiva, Hormone, Wirkstoffe zur Behandlung von Wunden und insbesondere chronischen Wunden, Vitamine, Pflanzenextrakte oder Auszüge aus Pflanzenextrakten, Psychopharmaka, den Schlaf beeinflussende Wirkstoffe, Analeptika, Allgemeinanästhetika, Muskelrelaxantien, Antiepileptika, Antiparkinsonmittel, Antiemetika, Ganglionär angreifende Substanzen, am Symphatikus angreifende Substanzen, am Parasamphatikus angreifende Substanzen, Calciumantagonisten, Herz-/Kreislaufmittel, Antiasthmatika, Antitussiva, Expektorantien, Hepatika, Diuretika, Choleretika, Desinfektionsmittel, Spurenelemente, Antiinfaktiva, Zytostatika, Antimetaboliten, Hormonanatgonisten und/oder Immunmodulatoren umfaßt.

14. Zubereitung nach einem der Ansprüche 1 bis 12, **dadurch gekennzeichnet, daß** die Zubereitung zur Anwendung bei Pflanzen als Wirkstoff ein Fungizid, ein Insektizid, ein Herbizid und/oder ein über Blatt- und/oder oberirdischen Pflanzenteilen aufnehmbares Düngemittel aufweist.

15. Zubereitung nach einem der vorangehenden Ansprüche, **dadurch gekennzeichnet, daß** die Zubereitung eine flüssige bis pastöse Viskosität zur topischen Applikation aufweist, wobei die Viskosität der Zubereitung vorzugsweise zwischen 0,4 cSt und 10.000 cSt, insbesondere zwischen 0,6 cSt und 1.500 cSt, variiert.

16. Zubereitung nach Anspruch 15, **dadurch gekennzeichnet, daß** die Zubereitung die siliciumorganische Verbindung und das mindestens eine Phospholipid in einem solch abgestimmten Massenverhältnis aufweist, das nach Auftragen der Zubereitung auf die menschliche bzw. tierische Haut in einer Konzentration zwischen 0,05 g und 0,3 g, jeweils pro 100 cm² Haut, innerhalb einer Zeit von 1 Sekunde bis 60 Sekunden, vorzugsweise innerhalb einer Zeit von 3 Sekunden bis 20 Sekunden, keine sichtbaren Rückstände der Zubereitung auf der Haut mehr verbleiben.

17. Zubereitung nach einem der vorangehenden Ansprüche, **dadurch gekennzeichnet, daß** die Zubereitung als pharmazeutischen und/oder kosmetischen Wirkstoff mindestens ein Vitamin aufweist.

18. Zubereitung nach Anspruch 17, **dadurch gekennzeichnet, daß** das Vitamin ein Vitamin C, Vitamin E, Vitamin A und/oder Pro-Vitamin A ist.

19. Zubereitung nach einem der vorangehenden Ansprüche, **dadurch gekennzeichnet, daß** die Zubereitung eine Darreichungsform besitzt, die eine Gabe der Zubereitung als Kapsel, Zäpfchen, Pflaster, Spray, Nebel, Gel, Puder und/oder Creme ermöglicht.

20. Zubereitung nach einem der vorangehenden Ansprüche, **dadurch gekennzeichnet, daß** sie zur topischen Applikation von pharmazeutischen und/oder kosmetischen Wirkstoffen, zur buccalen Applikation dieser Wirkstoffe und/oder zur oralen Applikation dieser Wirkstoffe aufgemacht ist.

## Claims

1. A pharmaceutical and/or cosmetic composition for use on humans, animals or plants, comprising at least one pharmaceutical and/or cosmetic active ingredient, at least one phospholipid and at least one organosilicon compound based on oligomeric or polymeric diorganosiloxane, **characterized in that**
a) the composition contains between 20 % by weight and 85 % by weight of the organosilicon compound and between 5 % by weight and 20 % by weight of the at least one phospholipid,
b) that the composition comprises as the organosilicon compound a cyclic oligomeric siloxane compound of the general formula I
(R₁-SiO-R₂)ₙ Formel I
and/or a linear siloxane compound of general formula II 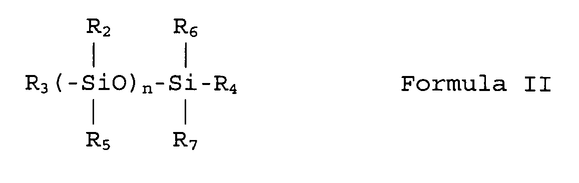 whereby in both formulas I and II
R₁, R₂, R₃, R₄, R₅, R₆ and/or R₇ may be the same or different and each may denote a C₁-C₄-alkyl group, preferably a saturated C₁-C₄-alkyl group, and/or
R₃ and/or R₄, R₆ and/or R₇ may be the same or different and may denote hydrogen and/or a hydroxyl group and
n is an integer between 0 and 30, and
c) that the composition contains such a phospholipid, having more than 70 % by weight of phosphatidylcholine.

2. The composition according to claim 1, **characterized in that** n is an integer between 0 and 8.

3. The composition according to one of the preceding claims, **characterized in that** the composition comprises such an organosilicon compound whose vapor pressure varies between 1 kPa and 7 kPa, preferably between 3,5 kPa and 5,7 kPa, and whose boiling point is between 15 °C and 150 °C.

4. The composition according to claim 1, wherein the composition comprises as the organosilicon compound the cyclic tetramer of general formula (I), whereby in formula I, n is equal to 4, and R₁and R₂ each denote a CH₃-group,
the cyclic pentamer of general formula I, whereby in formula I, n is equal to 5, and R₁and R₂each denote a CH₃-group, and/or
a linear siloxane of general formula II, whereby in formula II, n is equal to 2, 3 or 4, and R₂, R₃, R₄, R₅, R₆ and R₇ each denote a ch₃-group.

5. The composition according to one of the preceding claims, **characterized in that** the composition comprises as the phospholipid such a phospholipid, being isolated from natural starting materials.

6. The composition according to claim 5, **characterized in that** the phospholipid contains at least 80 % by weight and preferably more than 90 % by weight phosphatidylcholine.

7. The composition according to one of the preceding claims, **characterized in that** the phospholipid is a hydrogenated phospholipid, preferably a hydrogenated phosphatidylcholine.

8. The composition according to one of the preceding claims, **characterized in that** the composition comprises between 0 % by weight and 5 % by weight water, and the organosilicon compound and the phospholipid contained in the composition are mixed together so that the composition forms liposomes and/or micelles spontaneously on coming in contact with water.

9. The composition according to claim 8, **characterized in that** the liposomes and/or micelles formed spontaneously by adding water have an average particle size between 50 nm and 4000 nm, in particular between 250 nm and 1500 nm.

10. The composition according to one of the preceding claims, **characterized in that** the composition also comprises water, alcohol, antioxidants, non-phospholipid emulsifiers, pharmaceutical active ingredients, cosmetic active ingredients and/or other auxiliary substances, in addition to the at least one organosilicon compound and the at least one phospholipid.

11. The composition according to claim 10, **characterized in that** the concentration of the at least one pharmaceutical active ingredient and/or the at least one cosmetic active ingredient in the composition varies between 0,001 % by weight and 30 % by weight, in particular between 0,1 % by weight and 15 % by weight, based on the ready-to-use composition.

12. The composition according to one of the preceding claims, **characterized in that** the composition also contains at least one alcohol, where the concentration of the at least one alcohol amounts to a maximum of 14 % by weight and preferably varies between 0,1 % by weight and 8,5 % by weight, each based on the ready-to-use composition.

13. The composition according to one of the preceding claims, **characterized in that** the composition for use on humans or animals comprises at least one cosmetic and/or pharmaceutical active ingredient which is selected from the group consisting of local anesthetics, antiallergics, dermatologics, active ingredients to combat influenza infections and colds, active ingredients for treatment of neuropathies, active ingredients for treatment of circulation disorders, chemotherapeutics, quinine, antimycotics, antibiotics, thalidomide, analgesics, non-steroidal anti-inflammatory drugs, leukotrienes, leukotriene inhibitors, antiandrogens, corticosteroids, opiate receptor antagonists, blood coagulation inhibitors, platelet aggregation inhibitors, histamine antagonists, regulatory and enzymatic peptides and proteins, nucleic acids and oligopeptides, antidiabetics, prostaglandins, prostaglandin synthesis inhibitors, antiviral or virustatic substances, antimicrobial substances, active ingredients against prions, immuno-suppressants, hormones, active ingredients for treatment of wounds and especially chronic wounds, vitamins, plant extracts or extracts of plant extracts, psycho-pharmaceuticals, active ingredients to influence sleep, analeptics, general anesthetics, muscle relaxants, antiepileptics, antiparkinsonian drugs, antiemetics, substances that act on the ganglia, substances that act on the sympathetic nervous system, substances that act on the parasympathetic nervous system, calcium antagonists, cardiovascular agents, antiasthmatics, antitussives, expectorants, hepatics, diuretics, choleretics, disinfectants, trace elements, anti-infectives, cytostatics, antimetabolites, hormone antagonists and/or immunomodulators.

14. The composition according to one of the claims 1 to 12, **characterized in that** the composition for use on plants contains as the active ingredient a fungicide, an insecticide, a herbicide and/or a fertilizer absorbable through leaf parts and/or above-ground plant parts.

15. The composition according to one of the preceding claims, **characterized in that** the composition has a liquid to pasty viscosity for topical application, whereby the viscosity of the composition preferably varies between 0,4 cSt and 10.000 cSt, in particular between 0,6 cSt and 1500 cSt.

16. The composition according to claim 15, **characterized in that** the composition comprises the organosilicon compound and the at least one phospholipid in such a coordinated weight ratio that after applying the composition to human or animal skin in a concentration between 0,05 and 0,3 g per 100 cm² skin, no visible residues of the composition remain on the skin within a period of 1 second to 60 seconds, preferably within a period of 3 seconds to 20 seconds.

17. The composition according to one of the preceding claims, **characterized in that** the composition comprises at least one vitamin as the pharmaceutical and/or cosmetic active ingredient.

18. The composition according to claim 17, **characterized in that** the vitamin is vitamin C, vitamin E, vitamin A and/or pro-vitamin A.

19. The composition according to one of the preceding claims, **characterized in that** the composition is in such a form of administration which permits administration of the composition as a capsule, suppository, plaster, spray, mist, gel, powder and/or cream.

20. The composition according to one of the preceding claims, **characterized in that** it is prepared for topical application of pharmaceutical and/or cosmetic active ingredients, for buccal application of these active ingredients and/or for oral administration of these active ingredients.

## Revendications

1. Préparation pharmaceutique et/ou cosmétique pour une utilisation chez l'être humain, l'animal ou sur des plantes, comprenant au moins une substance active pharmaceutique et/ou cosmétique, au moins un phospholipide et au moins un composé organique de silicium à base d'un diorganosiloxane oligomère et/ou polymère, **caractérisée en ce que**
a) la préparation contient entre 20 % en poids et 85 % en poids du composé organique de silicium et entre 5 % en poids et 20 % en poids du au moins un phospholipide,
b) la préparation présente, comme composé organique de silicium, un composé de siloxane oligomère cyclique de formule générale I suivants :
(R₁-SiO-R₂)ₙ Formel I
et/ou un composé de siloxane linéaire de formule générale II suivante : 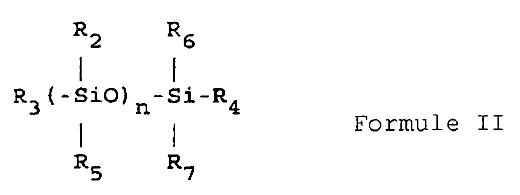 où, dans les deux formules I et II,
R₁, R₂, R₃, R₄, R₅, R₆ et/ou R₇ sont respectivement
identiques ou différents et représentent un groupement alkyle en C₁-C₄, de préférence, un groupement alkyle en C₁-C₄ saturé, et/ou R₃ et/ou R₄, R₆ et/ou R₇ sont identiques ou différents et
représentent l'hydrogène et/ou un groupement hydroxyle, et
n est un nombre entier compris entre 0 et 30, et **en ce que**
c) la préparation présente le genre de phospholipide qui possède plus de 70 % en poids de phosphatidylcholine.

2. Préparation selon la revendication 1, **caractérisée en ce que** n représente un nombre entier compris entre 0 et 8.

3. Préparation selon l'une quelconque des revendications précédentes, **caractérisée en ce que** la préparation présente le genre de composé organique de silicium dont la pression de vapeur varie entre 1 kPa et 7 kPa, de préférence, entre 3,5 kPa et 5,7 kPa, et dont le point d'ébullition varie entre 15 °C et 150 °C.

4. Préparation selon la revendication 1, **caractérisée en ce que** la préparation présente, comme composé organique de silicium, le tétramère cyclique de formule générale I, où, dans la formule I, n est égal à 4 et R₁ et R₂ représentent respectivement un groupement CH₃,
le pentamère cyclique de formule générale I, où, dans la formule I, n est égal à 5 et R₁ et R₂ représentent respectivement un groupement CH₃, et/ou un siloxane linéaire de formule générale II, où, dans la formule II, n est égal à 2, 3 ou 4 et R₂, R₃, R₄, R₅, R₆ et R₇ représentent respectivement un groupement CH₃.

5. Préparation selon l'une quelconque des revendications précédentes, **caractérisée en ce que** la préparation présente, comme phospholipide, un phospholipide isolé à partir d'une substance de départ naturelle.

6. Préparation selon la revendication 5, **caractérisée en ce que** le phospholipide contient au moins 80 % en poids et, de préférence, plus de 90 % en poids de phosphatidylcholine.

7. Préparation selon l'une quelconque des revendications précédentes, **caractérisée en ce que** le phospholipide est un phospholipide hydrogéné, de préférence, une phosphatidylcholine hydrogénée.

8. Préparation selon l'une quelconque des revendications précédentes, **caractérisée en ce que** la préparation présente entre 0 % en poids et 5 % en poids d'eau et **en ce que** le composé organique de silicium contenu dans la préparation et le phospholipide sont mélangés l'un à l'autre de sorte que la préparation forme, par contact avec l'eau, des liposomes et/ou des micelles spontanées.

9. Préparation selon la revendication 8, **caractérisée en ce que** les liposomes ou les micelles se formant spontanément par addition d'eau présentent une taille particulaire moyenne comprise entre 50 nm et 4000 nm, en particulier, entre 250 nm et 1500 nm.

10. Préparation selon l'une quelconque des revendications précédentes, **caractérisée en ce que** la préparation présente, outre le au moins un composé organique de silicium et le au moins un phospholipide, par ailleurs de l'eau, un alcool, des antioxydants, des émulsionnants non phospholipidiques, des substances actives pharmaceutiques, des substances actives cosmétiques et/ou d'autres adjuvants.

11. Préparation selon la revendication 10, **caractérisée en ce que** la concentration de la au moins une substance active pharmaceutique et/ou de la au moins une substance active cosmétique dans la préparation varie entre 0,001 % en poids et 30 % en poids, en particulier, entre 0, 1 % en poids et 15 % en poids sur la base de la préparation prête à l'emploi.

12. Préparation selon l'une quelconque des revendications précédentes, **caractérisée en ce que** la préparation contient en outre au moins un alcool, la concentration du au moins un alcool étant d'au maximum 14 % en poids et varie, de préférence, entre 0, 1 % en poids et 8,5 % en poids, respectivement, sur la base de la préparation prête à l'emploi.

13. Préparation selon l'une quelconque des revendications précédentes, **caractérisée en ce que** la préparation pour une utilisation chez l'être humain ou chez l'animal présente au moins une substance active cosmétique et/ou pharmaceutique, qui est choisie dans le groupe constitué des anesthésiques locaux, des antiallergiques, des produits dermatologiques, des substances actives luttant contre les infections grippales et les refroidissements, des substances actives pour le traitement de neuropathies, des substances actives pour le traitement de troubles de l'irrigation sanguine, des chimiothérapeutiques, de la quinine, des antimycotiques, des antibiotiques, du thalidomide, des agents analgésiques, des agents antirhumatismaux non stéroïdiens, des leucotriènes, des inhibiteurs de leucotriènes, des antiandrogènes, des corticoïdes, des antagonistes des récepteurs des opiacés, des substances inhibant la coagulation sanguine, des inhibiteurs de l'agrégation thrombocytaire, des antagonistes de l'histamine, des peptides et des protéines à activité régulatrice et enzymatique, des acides nucléiques et des oligopeptides, des antidiabétiques, des prostaglandines, des inhibiteurs de la synthèse des prostaglandines, des substances à activité antivirale ou à activité virostatique, des substances à activité antimicrobienne, des substances actives contre les prions, des immunosuppresseurs, des hormones, des substances actives pour le soin des plaies et, en particulier, des plaies chroniques, des vitamines, des extraits végétaux ou des préparations issues d'extraits végétaux, des psychotropes, des substances actives influençant le sommeil, des analeptiques, des anesthésiques généraux, des myorelaxants, des anti-épileptiques, des agents antiparkisoniens, des anti-émétiques, des substances affectant le système ganglionnaire, des substances affectant le système sympathique, des substances affectant le système parasympathique, des antagonistes du calcium, des agents cardiovasculaires, des anti-asthmatiques, des antitussifs, des expectorants, des agents hépatiques, des diurétiques, des cholérétiques, des agents désinfectants, des oligoéléments, des agents anti-infectieux, des cytostatiques, des antimétabolites, des antagonistes d'hormones et/ou des immunomodulateurs.

14. Préparation selon l'une quelconque des revendications 1 à 12, **caractérisée en ce que** la préparation pour une utilisation sur des plantes présente, comme substance active, un fongicide, un insecticide, un herbicide et/ou un engrais absorbable par les parties de feuille et/ou par les parties végétales aériennes.

15. Préparation selon l'une quelconque des revendications précédentes, **caractérisée en ce que** la préparation présente une viscosité liquide à pâteuse pour une application topique, la viscosité de la préparation variant, de préférence, entre 0,4 cSt et 10 000 cSt, en particulier, entre 0,6 cSt et 1500 cSt.

16. Préparation selon la revendication 15, **caractérisée en ce que** la préparation présente le composé organique de silicium et le au moins un phospholipide dans un rapport massique ajusté tel que, après application de la préparation sur la peau humaine ou animale en concentration comprise entre 0,05 g et 0,3 g, respectivement par 100 cm² de peau, sur une période de 1 seconde à 60 secondes, de préférence, sur une période de 3 secondes à 20 secondes, il ne reste plus aucun résidu visible de la préparation sur la peau.

17. Préparation selon l'une quelconque des revendications précédentes, **caractérisée en ce que** la préparation présente au moins une vitamine comme substances actives pharmaceutiques et/ou cosmétiques.

18. Préparation selon la revendication 17, **caractérisée en ce que** la vitamine est la vitamine C, la vitamine E, la vitamine A et/ou la provitamine A.

19. Préparation selon l'une quelconque des revendications précédentes, **caractérisée en ce que** la préparation possède une forme d'administration permettant une prise de la préparation sous la forme d'une gélule, d'un suppositoire, d'un timbre, d'un spray, d'un brouillard, d'un gel, d'une poudre et/ou d'une crème.

20. Préparation selon l'une quelconque des revendications précédentes, **caractérisée en ce qu'**elle est présentée pour une application topique de substances actives pharmaceutiques et/ou cosmétiques, pour une application buccale de ces substances actives et/ou pour une application orale de ces substances actives.
